# EUROPEAN PATENT SPECIFICATION

(11) **EP 1 311 699 B1**
(45) Date of publication and mention of the grant of the patent: **04.07.2007**
(21) Application number: 01974582.7
(22) Date of filing: 17.08.2001
(51) Int. Cl.: C12N 15/864, C12N 15/12, C07K 14/50, C07K 14/52, C07K 14/515, A61K 48/00

(54) **ADENO-ASSOCIATED VIRUS-MEDIATED DELIVERY OF ANGIOGENIC FACTORS**
ADENO-ASSOZIIERTE VIRUS-VERMITTELTE ÜBERTRAGUNG VON ANGIOGENESEFAKTOREN
DISTRIBUTION DE FACTEURS ANGIOGENIQUES A MEDIATION ASSUREE PAR VIRUS ASSOCIE AUX ADENOVIRUS

(30) Priority: 17.08.2000 US 226056 P
(43) Date of publication of application: 21.05.2003
(73) Proprietor: Ozawa, Keiya, Kawachi-gun, Tochigi 329-0498 (JP); Shimpo, Masahisa, Kawachi-gun, Tochigi 329-0498 (JP); Ikeda, Uichi, Kawachi-gun, Tochigi 329-0498 (JP); Maeda, Yoshikazu, Kawachi-gun, Tochigi 329-0498 (JP); Shimada, Kazuyuki, Kawachi-gun, Tochigi 329-0498 (JP)
(72) Inventor: Ozawa, Keiya, Kawachi-gun, Tochigi 329-0498 (JP); Shimpo, Masahisa, Kawachi-gun, Tochigi 329-0498 (JP); Ikeda, Uichi, Kawachi-gun, Tochigi 329-0498 (JP); Maeda, Yoshikazu, Kawachi-gun, Tochigi 329-0498 (JP); Shimada, Kazuyuki, Kawachi-gun, Tochigi 329-0498 (JP)
(74) Representative: HOFFMANN EITLE
(86) International application number: PCT/IB2001/001902
(87) International publication number: WO 2002/014487

(56) References cited:
- EP-A- 1 016 726
- WO-A-00/38518
- WO-A-97/12050
- WO-A-97/32990
- GOWDAK L. H. W. ET AL.: "Induction of angiogenesis by cationic lipid-mediated VEGF165 gene transfer in the rabbit ischemic hindlimb model." JOURNAL OF VASCULAR SURGERY, vol. 32, no. 2, 1 August 2000 (2000-08-01), pages 343-352, XP000949376 ISSN: 0741-5214
- FISHER K. J. ET AL.: "RECOMBINANT ADENO-ASSOCIATED VIRUS FOR MUSCLE DIRECTED GENE THERAPY" NATURE MEDICINE, vol. 3, no. 3, 1 March 1997 (1997-03-01), pages 306-312, XP000619697 ISSN: 1078-8956
- SU H. ET AL.: "Adeno-associated viral vector-mediated vascular endothelial growth factor gene transfer induces neovascular formation in ischemic heart." PROCEEDINGS OF THE NATIONAL ACADEMY OF SCIENCES OF THE UNITED STATES, vol. 97, no. 25, 5 December 2000 (2000-12-05), pages 13801-13806, XP002221628 ISSN: 0027-8424
- SHIMPO M. ET AL.: "AAV-mediated VEGF gene transfer into skeletal muscle stimulates angiogenesis and improves blood flow in a rat hindlimb ischemia model." CARDIOVASCULAR RESEARCH, vol. 53, no. 4, March 2002 (2002-03), pages 993-1001, XP002221629 ISSN: 0008-6363

### SEQUENCE LISTING

<110> OZAWA, Keiya
   SHIMPO, Masahisa
   IKEDA, Uichi
   MAEDA, Yoshikazu
   SHIMADA, Kazuyuki
<120> ADENO-ASSOCIATED VIRUS-MEDIATED DELIVERY OF ANGIOGENIC FACTORS
<130> 0800-0026.40
<140>
   <141>
<150> 60/226,056
   <151> 2000-08-17
<160> 2
<170> Patent In Ver. 2.0
<210> 1
   <211> 576
   <212> DNA
   <213> Artificial Sequence
<220>
   <223> Description of Artificial Sequence: VEGF-165
<220>
   <221> CDS
   <222> (1) .. (576)
<400> 1
   atg aac ttt ctg ctg tct tgg gtg cat tgg agc ctt gcc ttg ctg ctc     48
   Met Asn Phe Leu Leu Ser Trp Val His Trp Ser Leu Ala Leu Leu Leu
   1               5                   10                  15
   tac ctc cac cat gcc aag tgg tcc cag gct gca ccc atg gca gaa gga     96
   Tyr Leu His His Ala Lys Trp Ser Gln Ala Ala Pro Met Ala Glu Gly
               20                  25                  30
   gga ggg cag aat cat cac gaa gtg gtg aag ttc atg gat gtc tat cag     144
   Gly Gly Gln Asn His His Glu Val Val Lys Phe Met Asp Val Tyr Gln
           35                  40                  45
   cgc agc tac tgc cat cca atc gag acc ctg gtg gac atc ttc cag gag     192
   Arg Ser Tyr Cys His Pro Ile Glu Thr Leu Val Asp Ile Phe Gln Glu
       50                  55                  60
   tac cct gat gag atc gag tac atc ttc aag cca tcc tgt gtg ccc ctg     240
   Tyr Pro Asp Glu Ile Glu Tyr Ile Phe Lys Pro Ser Cys Val Pro Leu
   65                  70                  75                  80
   atg cga tgc ggg ggc tgc tgc aat gac gag ggc ctg gag tgt gtg ccc     288
   Met Arg Cys Gly Gly Cys Cys Asn Asp Glu Gly Leu Glu Cys Val Pro
                   85                  90                  95
   act gag gag tcc aac atc acc atg cag att atg cgg atc aaa cct cac     336
   Thr Glu Glu Ser Asn Ile Thr Met Gln Ile Met Arg Ile Lys Pro His
               100                 105                 110
   caa ggc cag cac ata gga gag atg agc ttc cta cag cac aac aaa tgt     384
   Gln Gly Gln His Ile Gly Glu Met Ser Phe Leu Gln His Asn Lys Cys
           115                 120                 125
   gaa tgc aga cca aag aaa gat aga gca aga caa gaa aat ccc tgt ggg     432
   Glu Cys Arg Pro Lys Lys Asp Arg Ala Arg Gln Glu Asn Pro Cys Gly
       130                 135                 140
   cct tgc tca gag cgg aga aag cat ttg ttt gta caa gat ccg cag acg     480
   Pro Cys Ser Glu Arg Arg Lys His Leu Phe Val Gln Asp Pro Gln Thr
   145                 150                 155                 160
   tgt aaa tgt tcc tgc aaa aac aca gac tcg cgt tgc aag gcg agg cag     528
   Cys Lys Cys Ser Cys Lys Asn Thr Asp Ser Arg Cys Lys Ala Arg Gln
                   165                 170                 175
   ctt gag tta aac gaa cgt act tgc aga tgt gac aag ccg agg cgg tga     576
   Leu Glu Leu Asn Glu Arg Thr Cys Arg Cys Asp Lys Pro Arg Arg
               180                 185                 190
<210> 2
   <211> 191
   <212> PRT
   <213> Artificial Sequence
<400> 2
   Met Asn Phe Leu Leu Ser Trp Val His Trp Ser Leu Ala Leu Leu Leu
   1               5                   10                  15
   Tyr Leu His His Ala Lys Trp Ser Gln Ala Ala Pro Met Ala Glu Gly
               20                  25                  30
   Gly Gly Gln Asn His His Glu Val Val Lys Phe Met Asp Val Tyr Gln
           35                  40                  45
   Arg Ser Tyr Cys His Pro Ile Glu Thr Leu Val Asp Ile Phe Gln Glu
       50                  55                  60
   Tyr Pro Asp Glu Ile Glu Tyr Ile Phe Lys Pro Ser Cys Val Pro Leu
   65                  70                  75                  80
   Met Arg Cys Gly Gly Cys Cys Asn Asp Glu Gly Leu Glu Cys Val Pro
                   85                  90                  95
   Thr Glu Glu Ser Asn Ile Thr Met Gln Ile Met Arg Ile Lys Pro His
               100                 105                 110
   Gln Gly Gln His Ile Gly Glu Met Ser Phe Leu Gln His Asn Lys Cys
           115                 120                 125
   Glu Cys Arg Pro Lys Lys Asp Arg Ala Arg Gln Glu Asn Pro Cys Gly
       130                 135                 140
   Pro Cys Ser Glu Arg Arg Lys His Leu Phe Val Gln Asp Pro Gln Thr
   145                 150                 155                 160
   Cys Lys Cys Ser Cys Lys Asn Thr Asp Ser Arg Cys Lys Ala Arg Gln
                   165                 170                 175
   Leu Glu Leu Asn Glu Arg Thr Cys Arg Cys Asp Lys Pro Arg Arg
               180                 185                 190

## Description

### FIELD OF THE INVENTION

The present invention relates to delivery of recombinant adeno-associated virus virions to muscle tissue. More specifically, the invention relates to the delivery of rAAV virions containing a transgene coding for an angiogenic factor to ischemic and non-ischemic muscle. The invention also relates to treating ischemic disease.

### BACKGROUND OF THE INVENTION

Coronary artery disease is the most common cause of heart failure in the Western world. In the United States, some 7 million people suffer from the affliction, with over 500,000 people dying from it each year, making coronary artery disease the number one killer of men and women in America. In addition, approximately 700,000 more Americans experience non-fatal heart attacks, with significant morbidity a common clinical outcome, as irreparable cardiac damage often ensues. This damage to cardiac tissue is caused by ischemia. Myocardial ischemia occurs when the cardiac muscle fails to receive an adequate blood supply and is thus deprived of essential levels of oxygen and nutrients. If the subsequent hypoxic and hypo-nutritional state is not corrected, cardiac tissue necrosis will occur; that is, a myocardial infarct will develop, and if severe enough, lead to cardiac arrest.

The most frequent cause of myocardial ischemia, atherosclerosis, results from a narrowing and hardening of the coronary arteries that provide blood flow to the cardiac muscle. The narrowing and hardening can become sufficiently relentless to completely block the affected artery. Other factors known to cause coronary arterial occlusion include thromboembolisms (in the absence of atherosclerosis) and congenital anatomical abnormalities.

Current therapeutic strategies for myocardial ischemia include pharmacological intervention, coronary artery bypass surgery, and non-surgical endovascular techniques (e.g., percutaneous transluminal angioplasty, endovascular stents). Standard pharmacological therapy is predicated on strategies that involve either increasing blood supply to the cardiac muscle or decreasing the demand of the cardiac muscle for oxygen and nutrients. Surgical treatment of ischemic heart disease is based on the bypass of diseased arterial segments with strategically placed bypass grafts. Non-surgical endovascular techniques are based on the use of catheters or stents to reduce the narrowing in diseased coronary arteries. All of these strategies are used to decrease the number of, or to eliminate, ischemic episodes, but all have various limitations. The need to develop more effective therapeutic strategies is partially based on the fact that surviving victims of ischemic episodes are at substantially greater risk for subsequent episodes of ischemia, which in many cases prove fatal.

In addition to the heart, ischemia may occur in the brain, peripheral limbs, lungs, and kidneys, leading to stroke, deep vein thrombosis, pulmonary embolus, and renal failure. Peripheral arterial disease (i.e., non-myocardial ischemia) affects approximately 8-10 million people in the United States. It frequently leads to peripheral neuropathies, where the sensory and motor neurons are adversely affected. The prognosis of patients with these risk factors is limited because of their greater risks for myocardial infarction, stroke, and cardiovascular death. Often it is necessary to treat both peripheral and myocardial ischemia together.

Many novel therapeutic strategies for treating ischemic disease are focused on stimulating the development of new blood vessels, a process known as angiogenesis. Angiogenesis, or the proliferation of new capillary blood vessels, is a fundamental process necessary for the normal growth and development of tissues. It is a requirement for the development and differentiation of the vascular tree, as well as for a wide variety of other physiological processes. Among these, angiogenesis occurs as part of the body's repair mechanisms, e.g., in the healing of wounds.

Capillary blood vessels consist of endothelial cells and pericytes. These two cell types carry the requisite genetic information to form tubes, branches and entire capillary networks. Specific molecules can initiate this process. In view of the physiological importance of angiogenesis, much effort has been devoted to the isolation, characterization and purification of factors that can stimulate angiogenesis, and a number of polypeptides that do so have been purified and characterized.

One such angiogenic factor, which specifically binds to and activates vascular endothelial cells, is vascular endothelial growth factor (VEGF). VEGF is a potent vasoactive protein. Four different molecular variants of VEGF have been described. The 165 amino acid variant is the predominant molecular form found in normal cells and tissues. A less abundant, shorter form with a deletion of 44 amino acids between positions 116 and 159 (VEGF₁₂₁), a longer form with an insertion of 24 basic residues in position 116 (VEGF₁₈₉), and another longer form with an insertion of 41 amino acids (VEGF₂₀₆), which includes the 24 amino acid insertion found in VEGF₁₈₉, are also known. VEGF₁₂₁ and VEGF₁₆₅ are soluble proteins. VEGF₁₈₉ and VEGF₂₀₆ appear to be mostly cell-associated. All of the versions of VEGF are biologically active.

The various forms of VEGF are encoded by the same gene and arise from alternative splicing of messenger RNA. This conclusion is supported by Southern blot analysis of human genomic DNA, which shows that the restriction pattern is identical using either a probe for VEGF₁₆₅ or one that contains the insertion in VEGF₂₀₆. Analysis of genomic clones in the area of putative mRNA splicing also shows an intron/exon structure consistent with alternative splicing. Recently, a new isoform of VEGF*, called VEGF-2, has been described. Its expression profile is similar to VEGF, and has been shown to stimulate the proliferation of vascular endothelial cells, while inhibiting the proliferation-stimulating effect on vascular smooth muscle cells elicited by platelet-derived growth factor.

VEGF can have diverse effects that depend on the specific biological context in which it is found. VEGF is a potent endothelial cell mitogen and directly contributes to induction of angiogenesis in vivo by promoting endothelial cell proliferation during normal development or during wound healing. A most striking property of VEGF is its specificity. It is mitogenic in vitro at 1 ng/mL for capillary and human umbilical vein endothelial cells, but not for adrenal cortex cells, corneal or lens epithelial cells, vascular smooth muscle cells, corneal endothelial cells, granulosa cells, keratinocytes, BHK-21 fibroblasts, 3T3 cells, rat embryo fibroblasts, human placental fibroblasts and human sarcoma cells. The target cell specificity of VEGF appears to be restricted to vascular endothelial cells. By binding to its receptor (an endothelial cell-surface tyrosine kinase receptor), VEGF can trigger the entire sequence of events leading to angiogenesis and has been shown to stimulate capillary growth in various animal models of ischemia. It is able to stimulate the proliferation of endothelial cells isolated from both small and large vessels. VEGF expression is triggered by hypoxemia so that endothelial cell proliferation and angiogenesis appear to be especially stimulated in ischemic areas.

Another angiogenic factor that has been relatively well characterized is fibroblast growth factor (FGF). There are at least nine members of the FGF family, namely FGF-1 (alternatively termed acidic FGF) through FGF-9, not all of which are associated with angiogenesis. FGF-2, also known as basic FGF, is one of the most extensively characterized proteins in the angiogenic process. For example, studies have shown that an injection of FGF-2 into adult canine coronary arteries during coronary occlusion leads to an increase in new blood vessel formation, reportedly leading to a decrease in myocardial dysfunction. Similar results have been reported in other animal models of myocardial ischemia. In cell-based assays, FGF-2 has been shown to have a synergistic effect with VEGF to induce endothelial cell proliferation into capillary-like structures. In vivo, FGF-2, along with VEGF, has been shown to induce angiogenesis. The FGF receptor is similar to the VEGF receptor in that it is a cell-surface tyrosine kinase receptor. Ligand binding activates the receptor by inducing a conformational change resulting in the triggering of the intrinsic tyrosine kinase activity, which leads to a signal transduction cascade that affects gene expression and ultimately results in endothelial cell proliferation.

Angiopoietin-1, a recently discovered angiogenic factor, was first identified by its involvement in the later stages of angiogenesis. For example, studies have shown that, when VEGF and angiopoietin-1 are introduced into an angiogenic assay together, larger, more numerous, and more highly branched vessels formed relative to VEGF treatment alone. Another study showed that angiopoietin-1 induced endothelial cell proliferation and capillary growth in the absence of VEGF, and that these new blood vessels were not "leaky," unlike VEGF, which induced the formation of new blood vessels growth that were leaky. The same study demonstrated that coexpression of VEGF and angiopoietin-1 led to an additive effect of new blood vessel formation, and that these new blood vessels were not leaky.

A prerequisite for achieving an angiogenic effect with these proteins however, has been the need for repeated or long-term delivery of the protein, which limits the utility of directly injecting these proteins to stimulate angiogenesis in clinical settings. Therefore, an approach that does not rely on repeated injection or infusion of angiogenic factors, which would allow for long-term and sustained levels of angiogenic factor expression, and would achieve defined therapeutic endpoints, would provide potential benefits in the treatment of ischemic diseases. Several gene therapy methods are currently being developed to achieve this end.

Ideally, such gene therapy methods will permit the delivery of sustained levels of specific proteins (or other therapeutic molecules) to the patient. A nucleic acid molecule can be introduced directly into a patient (*in vivo* gene therapy), or into cells isolated from a patient or a donor, which are then subsequently returned to the patient (*ex vivo* gene therapy). The introduced nucleic acid then directs the patient's own cells or grafted cells to produce the desired therapeutic product. Gene therapy may also allow clinicians to select specific organs or cellular targets (e.g., muscle, blood cells, brain cells, etc.) for therapy.

Nucleic acids may be introduced into a patient's cells in several ways, including viral-mediated gene delivery, naked DNA delivery, and transfection methods. Viral-mediated gene delivery has been used in a majority of gene therapy trials. The recombinant viruses most commonly used in gene therapy trials (as well as pre-clinical research) are those based on retrovirus, adenovirus, herpes virus, pox virus, and adeno-associated virus (AAV). Alternatively, transfection methods may be used for gene delivery. Although transfection methods are generally not suitable for *in vivo* gene delivery, they may be utilized for *ex vivo* gene transfer. Such methods include chemical transfection methods, such as calcium phosphate precipitation and liposome-mediated transfection, as well as physical transfection methods such as electroporation.

### AAV-Mediated Gene Therapy

AAV, a parvovirus belonging to the genus Dependovirus, has several features not found in other viruses. These features make it particularly well suited for gene therapy applications. For example, AAV can infect a wide range of host cells, including non-dividing cells. Furthermore, AAV can infect cells from a variety of species. Importantly, AAV has not been associated with any human or animal disease, and does not appear to alter the physiological properties of the host cell upon transduction. Finally, AAV is stable at a wide range of physical and chemical conditions, which lends itself to production, storage, and transportation requirements.

The AAV genome, a linear, single-stranded DNA molecule containing approximately 4700 nucleotides (the AAV-2 genome consists of 4681 nucleotides, the AAV-4 genome 4767), generally comprises an internal non-repeating segment flanked on each end by inverted terminal repeats (ITRs). The ITRs are approximately 145 nucleotides in length (AAV-1 has ITRs of 143 nucleotides) and have multiple functions, including serving as origins of replication, and as packaging signals for the viral genome.

The internal non-repeated portion of the genome includes two large open reading frames (ORFs), known as the AAV replication (rep) and capsid (cap) regions. These ORFs encode replication and capsid gene products; which allow for the replication, assembly, and packaging of a complete AAV virion. More specifically, a family of at least four viral proteins are expressed from the AAV *rep* region: Rep 78, Rep 68, Rep 52, and Rep 40, all of which are named for their apparent molecular weights. The AAV *cap* region encodes at least three proteins: VP1, VP2, and VP3.

AAV is a helper-dependent virus, that is, it requires co-infection with a helper virus (e.g., adenovirus, herpesvirus, or vaccinia virus) in order to form functionally complete AAV virions. In the absence of co-infection with a helper virus, AAV establishes a latent state in which the viral genome inserts into a host cell chromosome or exists in an episomal form, but infectious virions are not produced. Subsequent infection by a helper virus "rescues" the integrated genome, allowing it to be replicated and packaged into viral capsids, thereby reconstituting the infectious virion. While AAV can infect cells from different species, the helper virus must be of the same species as the host cell. Thus, for example, human AAV will replicate in canine cells that have been co-infected with a canine adenovirus.

To produce infectious recombinant AAV (rAAV) containing a heterologous nucleic acid sequence, a suitable host cell line can be transfected with an AAV vector containing the heterologous nucleic acid sequence, but lacking the AAV helper function genes, *rep* and *cap.* The AAV-helper function genes can then be provided on a separate vector. The helper virus genes necessary for AAV production (i.e., the accessory function genes) can be provided on a vector, rather than providing a replication-competent helper virus (such as adenovirus, herpesvirus, or vaccinia). Alternatively, AAV helper functions can be incorporated into a helper virus genome, preferably one that has been made replication-incompetent. For example, AAV *cap* can be incorporated into an adenoviral genome such as adenovirus serotype 5, and the adenovirus can then infect host cells and provide the Cap protein helper function as well as the necessary helper virus accessory functions.

Collectively, the AAV helper function genes (i.e., *rep* and *cap)* and accessory function genes can be provided on one or more vectors. Helper and accessory function gene products can then be expressed in the host cell where they will act in *trans* on rAAV vectors containing the heterologous nucleic acid sequence. The rAAV vector containing the heterologous nucleic acid sequence will then be replicated and packaged as though it were a wild-type (wt) AAV genome, forming a recombinant virion. When a patient's cells are infected with the resulting rAAV virions, the heterologous nucleic acid sequence enters and is expressed in the patient's cells. Because the patient's cells lack the *rep* and *cap* genes, as well as the accessory function genes, the rAAV cannot further replicate and package their genomes. Moreover, without a source of *rep* and *cap* genes, wtAAV cannot be formed in the patient's cells.

There are six known. AAV serotypes, AAV-1 through AAV-6. AAV-2 is the most prevalent serotype in human populations; one study estimated that at least 80% of the general population has been infected with wtAAV-2. AAV-3 and AAV-5 are also prevalent in human populations, with infection rates of up to 60%. AAV-1 and AAV-4 are simian isolates, although both serotypes can transduce human cells. Of the six known serotypes, AAV-2 is the best characterized. For instance, AAV-2 has been used in a broad array of *in vivo* transduction experiments, and has been shown to transduce many different tissue types. Investigators have exploited the broad tissue tropism of AAV-2 to deliver many different tissue-specific transgenes.

The need to develop new therapeutic strategies for treating various ischemic diseases is evident by the number of individuals afflicted with such disorders. Coronary artery disease developing into ischemia and subsequent myocardial infarct is the leading cause of death among adults in the Western world. Stimulating new blood vessel growth (angiogenesis) to deliver increased blood flow to oxygen-starved tissue is one such novel therapeutic strategy to reduce the number of myocardial infarctions. To overcome the inherent limitations in current experimental techniques used for stimulating angiogenesis (e.g., transient low-level expression with plasmid DNA, or potential inflammation with adenovirus), methods using AAV to deliver angiogenic factors to ischemic tissues have been developed and are described herein.

### SUMMARY OF THE INVENTION

In accordance with the present invention, a use is provided which allows for the efficient transfer of genes to muscle tissue using recombinant AAV virions. The methods result in the long-term and stable expression of the genes transferred.

Using rAAV virions that are lacking wild-type (wt) AAV virions and helper viruses (such as adenovirus) facilitates safe and efficient gene transfer. Accordingly, the invention broadly encompasses the use of rAAV virions, free of wt-AAV and adenovirus (or other helper viruses), to deliver genes encoding angiogenic factors to muscle tissue in order to produce a therapeutic effect namely treatment or prevention of coronary artery diseases and ischemic conditions. In one embodiment of the present invention, the angiogenic factor is VEGF. In a preferred embodiment, the VEGF is VEGF₁₆₅. In another embodiment, the angiogenic factor is FGF. In yet another embodiment, the angiogenic factor is angiopoietin-1. The therapeutic effect, in one aspect of the invention, is an increase in new blood vessel formation. In another aspect, the therapeutic effect is an increase in blood flow.

To facilitate the growth of new blood vessels and an increase in blood flow to a muscle, it is necessary to deliver the angiogenic factor gene to the muscle, and in such a manner that efficient gene expression is achieved. Accordingly, the rAAV virion delivery is by way of direct injection into muscle tissue. In one aspect, the muscle tissue is skeletal muscle tissue. In another aspect, the muscle tissue is cardiac muscle tissue. In yet another aspect, the muscle tissue is smooth muscle tissue.

So as to treat ischemic disease rAAV virions, free of wt-AAV and helper viruses and containing a gene coding for an angiogenic factor, are delivered to muscle tissue where the gene is expressed and a therapeutic effect is achieved. In one aspect, the angiogenic factor is VEGF, preferably VEGF₁₆₅. In another aspect, the angiogenic factor is FGF. In yet another aspect, the angiogenic factor is angiopoietin-1. The therapeutic effect, in one aspect, is an increase in new blood vessel formation. In another aspect, the therapeutic effect is an increase in blood flow to the ischemic muscle tissue.

Growth of new blood vessels and enhancement of blood flow to ischemic muscle is realized when angiogenic factor genes are delivered to an appropriate site, and accomplished in such a manner that efficient gene expression is achieved. Accordingly treating ischemia in a muscle is by way of direct injection of rAAV virions, containing a gene coding for an angiogenic factor, into the muscle experiencing ischemia. In one aspect, the muscle is a skeletal muscle. In another aspect, the muscle is a cardiac muscle. In yet another aspect, the muscle is a smooth muscle.

These and other embodiments of the subject invention will readily occur to those of ordinary skill in the art in view of the disclosure herein.

### BRIEF DESCRIPTION OF THE DRAWINGS

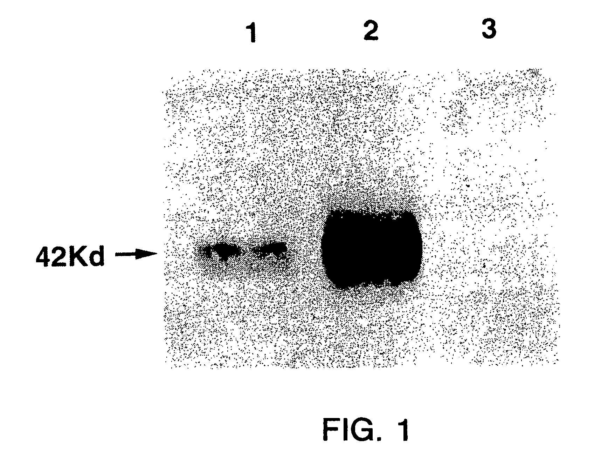

FIG. 1 shows an immunoblot of rAAV-hVEGF165-transduced rat cardiac myocytes. Cardiac myocytes were transduced with rAAV-hVEGF₁₆₅ or rAAV-LacZ (5 x 10³ virions/cell). Twenty-four hours after transduction, the cells were lysed. VEGF protein in the lysates was separated by 7.5% polyacrylamide gel electrophoresis and blotted onto membranes. Expression of 42-kDa VEGF protein was evident in the rAAV-hVEGF₁₆₅-transduced myocytes (lane 2), whereas no VEGF protein was detected in the rAAV-LacZ-transduced cardiac myocytes (lane 3). Lane 1 shows human recombinant VEGF₁₆₅ (3 ng/lane) and serves as a positive control.

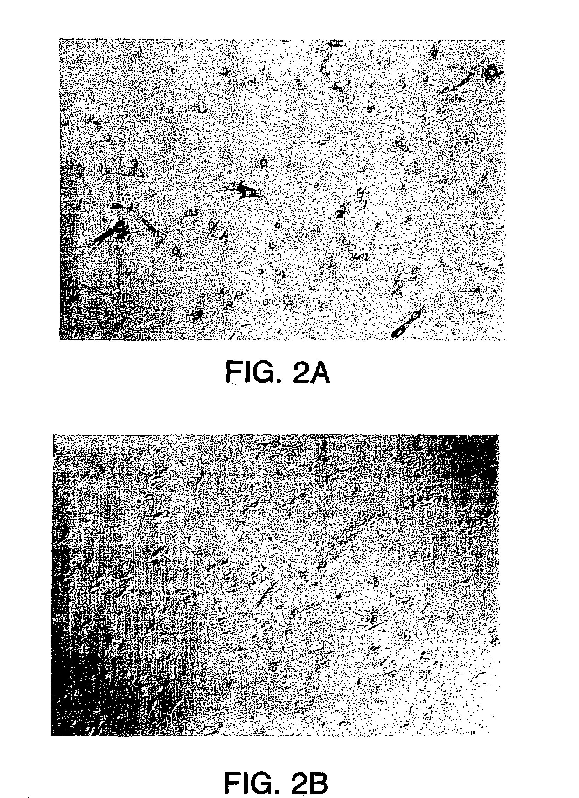

FIG. 2 shows the results of immunohistochemical staining for VEGF in transduced rat cardiac myocytes. Cells were transduced with rAAV-hVEGF₁₆₅ (A) or rAAV-LacZ (B) at 5 x 10³ virions/cell for 24 h followed by staining with anti-human VEGF165 antibody. Original magnification x100.

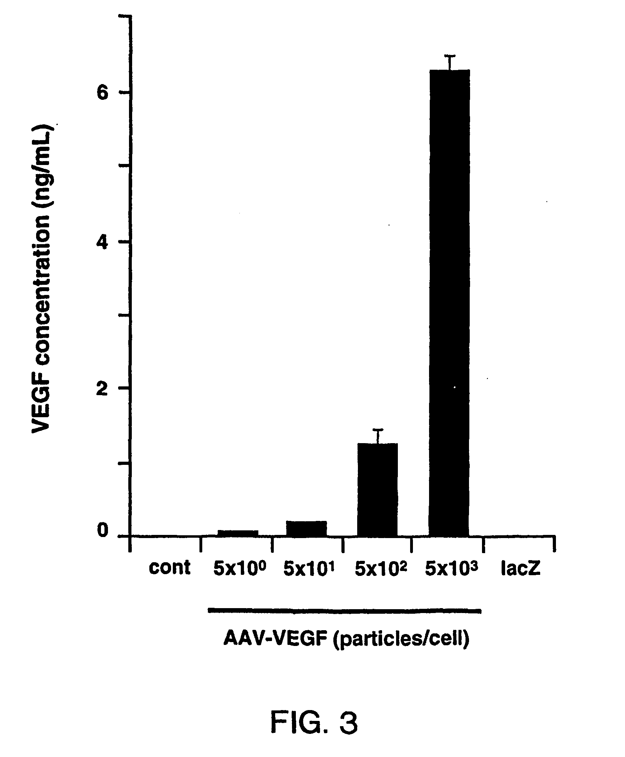

FIG. 3 shows VEGF concentration in the culture medium of rat cardiac myocytes. Myocytes were exposed to increasing doses of rAAV-hVEGF165 or rAAV-LacZ. Forty-eight hours after transduction, VEGF concentration was measured by ELISA. Data expressed as mean values ± SEM (n=4), and are representative of three different experiments.

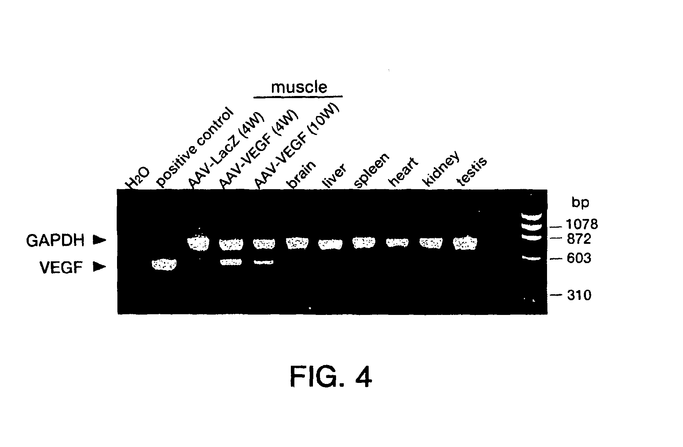

FIG. 4 shows expression of VEGF mRNA in vector-injected muscle tissues and organs. Muscle tissues and organs were isolated at various time points after intramuscular injection and total RNA was extracted. After DNase I treatment, RT-PCR using human VEGF-specific primers was performed. The sizes of PCR products for rat GAPDH and human VEGF were 747 bp and 531 bp, respectively. VEGF expression plasmid (pCMV-VEGF) was used as a positive control. GAPDH mRNA served as an internal standard. The PCR products were electrophoresed on ethidium bromide-stained 0.2% agarose gels. Three independent experiments yielded identical results.

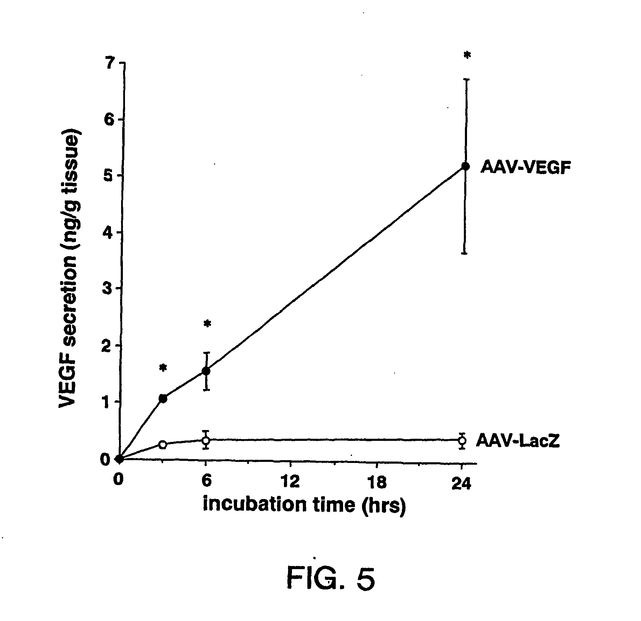

FIG. 5 depicts VEGF secretion from rAAV-hVEGF₁₆₅-injected tibialis anterior muscles. Ten weeks after injection, muscle tissues were excised and cultured in serum-free DMEM/F-12 medium. An ELISA kit was used to measure culture supernatant VEGF concentration. VEGF concentrations were normalized to the protein content per dish and are shown as mean values ± SEM of measurements from one experiment (n=4), representative of two different experiments (p<0.05 compared with values of AAV-LacZ-transduced muscles).

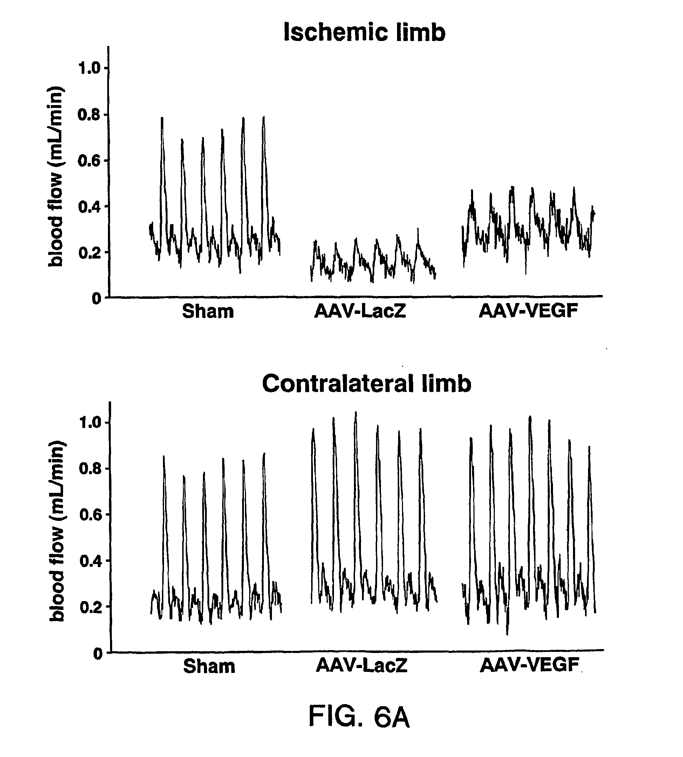

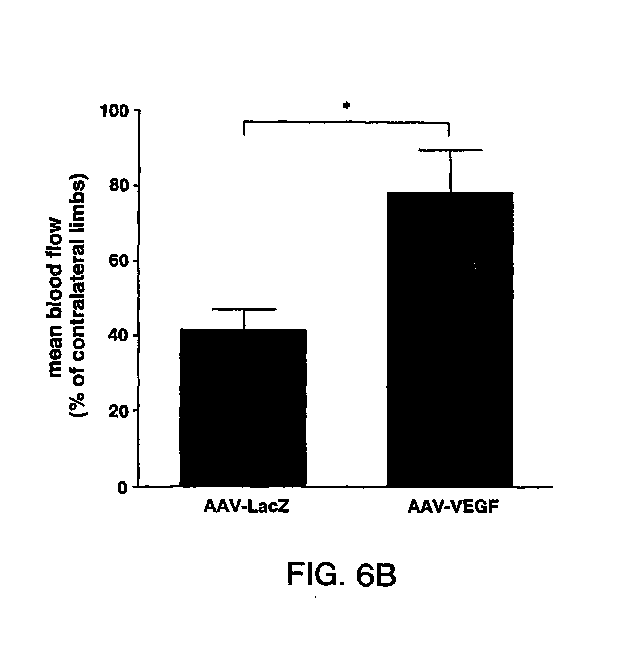

FIG. 6 depicts blood flow in the rat hindlimb as measured by transit-time ultrasound flowmeter 6 weeks after rAAV-hVEGF₁₆₅ injection. After anesthetization, blood flow of both ischemic and contralateral limbs was recorded simultaneously in sham-operated (n=3), AAV-LacZ-transduced (1.5 x 10¹³ particles/body; n=8) and rAAV-VEGF₁₆₅-transduced (2.0 x 10¹³ particles/body; n=8) rats. 6A: Representative record of blood flow. **6B:** Mean blood flow in the ischemic limbs. Values are expressed as % of contralateral limbs and are shown as mean values ± SEM (p<0.01)

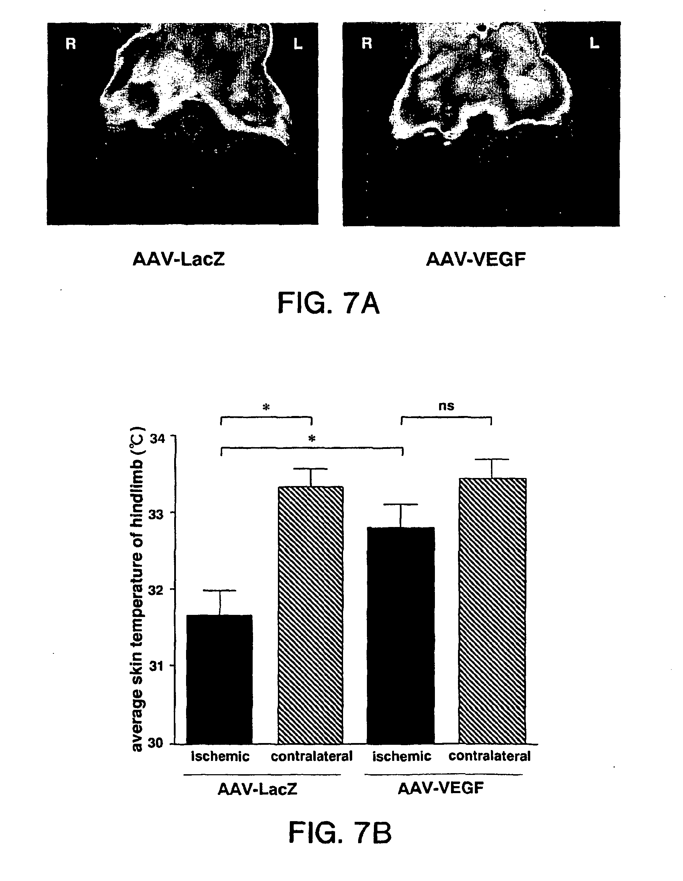

FIG. 7 depicts thermography of rat hindlimbs performed 6 weeks after rAAV-hVEGF₁₆₅ injection. Rats were anesthetized and their lower body coats were shaved. The skin temperature of each rat hindlimb was measured with infrared thermography. **7A**: Images of infrared thermography. R; ischemic limbs, L; contralateral limbs. **7B:** Average skin temperature (°C) of ischemic and contralateral hindlimbs in AAV-LacZ-(1.5 x10¹³ particles/body; n=4) or AAV-hVEGF₁₆₅- (2.0 x 10¹³ particles/body; n=4) transduced rats (p<0.05).

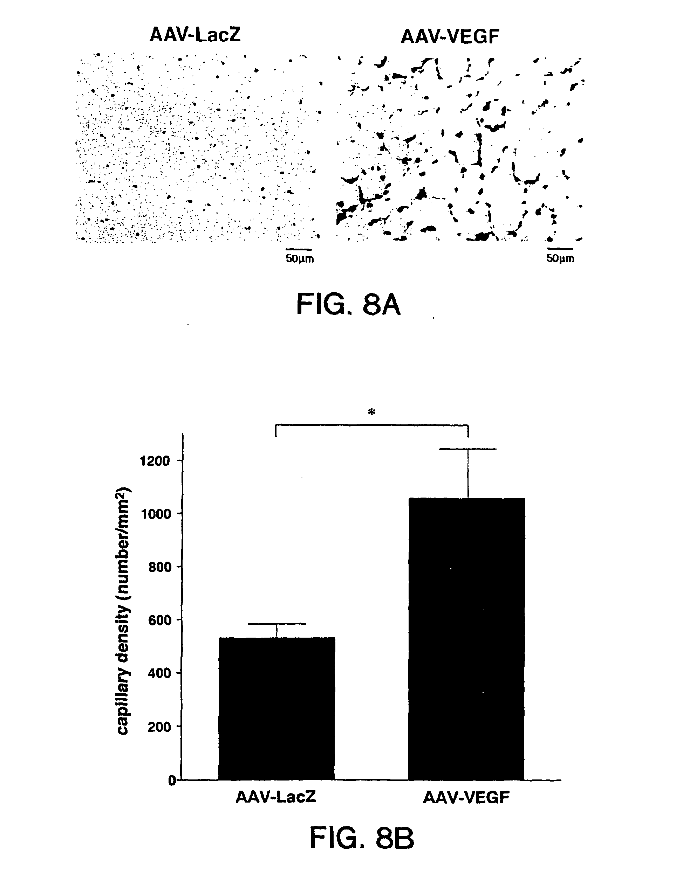

FIG. **8. 8A:** Representative images of rat muscle tissues using histochemical staining for alkaline phosphatase. **8B:** Capillary density in rat muscle tissues. Muscle tissues were obtained from the ischemic limbs of AAV-LacZ-transduced (n=4) and AAV-VEGF-transduced (n=4) rats. Capillary number was counted in 5 different fields of one muscle section, and capillary density was calculated. Data are shown as mean values ± SEM (p<0.001).

### DETAILED DESCRIPTION OF THE INVENTION

The invention provides methods for delivering recombinant AAV virions to a mammal, including a human. More particularly, it involves delivery of rAAV virions comprising a heterologous nucleic acid sequence encoding an angiogenic factor. By "recombinant AAV virion" or "rAAV virion" is meant an infectious virus composed of an AAV protein shell (i.e., a capsid) encapsulating a heterologous nucleic acid sequence that is flanked by one or more AAV ITRs. The "heterologous nucleic acid sequence" encapsulated includes nucleic acid sequences joined together that are not normally found in association with each other in nature. For example, a heterologous nucleic acid sequence could include a coding sequence flanked by sequences not found in association with the coding sequence in nature. Another example of a heterologous nucleic acid sequence is a coding sequence that is not found in nature (e.g., synthetic sequences having codons different from the native gene).

Preferably, recombinant AAV virions are produced using a triple transfection system that is described in U. S. Patent Nos. 6,001,650 and 6,004,797. This system involves the use of three vectors for rAAV virion production, including an accessory function vector, an AAV helper function vector, and a rAAV vector. Accessory function vectors, AAV helper function vectors, and recombinant AAV vectors can be engineered using conventional recombinant techniques. For example, nucleic acid molecules can be excised from a viral genome or a vector containing the same and readily assembled in any desired order by inserting one or more of the desired nucleotide sequences into a construct, such as those commercially available from Stratagene, La Jolla, Calif. and other sources which are well known to those of skill in the art.

The triple transfection method can make use of the pladeno5 accessory function vector (described in U. S. Patent No. 6,004,797), the AAV helper function vector pHLP19 (described in U. S. Patent No. 6,001,650), and a rAAV vector containing the heterologous nucleic acid sequence encoding an angiogenic factor. One of skill in the art will appreciate, however, that the nucleic acid sequences encoded by these vectors can be provided on two or more vectors in various combinations.

Alternatively, rAAV virions can be produced by incorporating AAV accessory function genes into a helper virus genome such as adenovirus serotype 5, preferably a helper virus that has been rendered replication-deficient, and more preferably one that has been rendered replication-incompetent. Once an AAV helper function gene, such as *cap,* has been incorporated into the genome of the helper virus, the helper virus, e.g., adenovirus, can then infect host cells and provide the AAV Cap protein helper function as well as the necessary helper virus accessory functions.

As used herein, the term "vector" includes any genetic element, such as a plasmid, phage, transposon, cosmid, chromosome, artificial chromosome, virus, virion, etc., which is capable of replication when associated with the proper control elements and which can transfer gene sequences between cells. Thus, the term includes cloning and expression vehicles, as well as viral vectors.

The AAV helper function vector encodes the "AAV helper function" sequences (i.e., *rep* and *cap*), which function *in trans* for productive AAV replication and encapsidation. As is described in U. S. Patent No. 6,001,650, the AAV helper function vector facilitates efficient rAAV virion production without generating any detectable wild-type or pseudo-wild-type AAV; consequently, the triple-transfection system will allow for the production of rAAV virions without generating any detectable wild-type or pseudo-wild-type AAV virions. By "detectable" is meant the detection of any wild-type or pseudo-wild-type AAV DNA using PCR (as described in U. S. Patent No. 6,001,650, supra) or equivalent technique having the same or similar sensitivity and specificity.

The accessory function vector encodes nucleotide sequences for non-AAV derived viral and/or cellular functions upon which AAV is dependent for replication (i.e., "accessory functions"). The accessory functions include those functions required for AAV replication, including, without limitation, those moieties involved in activation of AAV gene transcription, stage specific AAV mRNA splicing, AAV DNA replication, synthesis of Cap expression products, and AAV capsid assembly. Viral-based accessory functions can be derived from any of the known helper viruses such as adenovirus, herpesvirus (other than herpes simplex virus type-1), and vaccinia virus. As a result, the accessory function vector facilitates efficient AAV vector production without the use of any helper viruses, so rAAV virions generated using the triple-transfection system will be free of adenovirus, herpesvirus, poxvirus, vaccinia virus, etc.

The "AAV vector" can be a vector derived from any AAV serotype, including without limitation, AAV-1, AAV-2, AAV-3A, AAV-3B, AAV-4, AAV-5, AAV-6, etc. AAV vectors can have one or more of the wt-AAV genes deleted in whole or in part, i.e., the *rep* and/or *cap* genes, but retain at least one functional flanking ITR sequences, as necessary for the rescue, replication, and packaging of the AAV virion. Thus, an AAV vector is defined herein to include at least those sequences required in *cis* for viral replication and packaging (e.g., functional ITRs). The ITRs need not be the wild-type nucleotide sequences, and may be altered, e.g., by the insertion, deletion, or substitution of nucleotides, so long as the sequences provide for functional rescue, replication, and packaging. Furthermore, the AAV helper proteins, i.e., Rep and Cap, need not be derived from the same serotype as the ITRs. For example, by using well-known art techniques, one could construct a vector using the ITRs from wt-AAV-2, and provide helper functions (i.e., *rep* and *cap)* from wt-AAV-6, or *rep* from AAV-2 and *cap* from AAV-6. Alternatively, one could use altered ITRs based on the sequence of a particular wt-AAV serotype such as AAV-2, and helper functions from other AAV serotypes. One skilled in the art can appreciate the variety of possible combinations of ITRs and helper function proteins that can be used to construct intact and functional rAAV virions. AAV vectors can include one or more heterologous nucleic acid sequences flanked with functional AAV ITRs, the incorporation of the heterologous nucleic acid sequence defining a "rAAV vector."

Recombinant AAV virions can be purified using a variety of purification techniques that are well known in the art such as cesium chloride density centrifugation or column chromatography. Once purified, rAAV virions can be formulated into stable pharmaceutical compositions, for example as described in International Publication WO 00/32233, for delivery to a mammalian subject.

The invention contemplates the delivery of one or more therapeutic nucleotide sequences. In particular, the invention encompasses AAV vectors encoding any of the known angiogenic factors, which factors may be delivered, using the methods of the present invention, to muscle cells of a mammal, including those of a human. Thus, the invention encompasses: delivery of VEGF for the treatment of peripheral and myocardial ischemia, delivery of FGF for the treatment of peripheral and myocardial ischemia, and the delivery of angiopoietin-1 for the treatment of peripheral and myocardial ischemia. Each angiogenic factor can be delivered alone or in combination. For example, VEGF can be delivered alone to stimulate new blood vessel formation and enhance blood flow, or VEGF can be delivered in conjunction with FGF and/or angiopoietin-1 to stimulate new blood vessel formation and enhance blood flow.

Moreover, the invention contemplates delivery of any of the various active forms of these angiogenic factors. For example, as described above, four different biologically active splice variants of VEGF are known. See, e.g., Tischer et al., J. Biol. Chem. (1991) 266:11947-11954, describing the sequence of VEGF₁₆₅ (see, also, SEQ ID NOS:1 and 2 and GenBank Accession no. AB021221), VEGF₁₂₁ (see, also, GenBanlc Accession no. AF214570) and VEGF₁₈₉; and Houck et al., Mol. Endocrinol. (1991) 5:1806-1814, describing the sequence of VEGF₂₀₆. VEGF₁₆₅ has 165 amino acids and is the predominant molecular form found in normal cells and tissues. VEGF₁₂₁ is a less abundant, shorter form with a deletion of 44 amino acids between positions 116 and 159. VEGF₁₈₉ is a longer form with an insertion of 24 basic residues in position 116, and VEGF₂₀₆ is another longer form with an insertion of 41 amino acids, which includes the 24 amino acid insertion found in VEGF₁₈₉.

The present invention is also useful for purposes other than treating an ischemic condition or delivering a known angiogenic factor to a muscle. For example, an unknown gene that is thought to be an angiogenic factor gene can be expressed using the methods described herein. By way of example, it can be envisioned that bioinformatics discovers an unknown gene "X," which has a high degree (e.g., >70%) of nucleotide sequence identity with a known angiogenic factor gene "Y," sufficient to identify X as a putative angiogenic factor gene. In this case, the X gene could be cloned into an AAV vector, thereby creating a new vector rAAV-X and, using the present invention, rAAV-X could be expressed to ascertain whether new blood vessel formation and/or increased blood flow results from expression of X. The same can be done for Y to determine whether the functions of X and Y are the same or similar. Therefore, the present invention can be used to advance the field of functional genomics.

The present invention is also useful to establish a drug-screening assay, in a manner somewhat analogous to assays described in U. S. Patent Nos. 4,980,281 and 5,688,655. For example, if inhibitors to the angiogenic process were desired (e.g., in cancer research), the present invention could be used to deliver one or more angiogenic factors to a mammal, the angiogenic factor(s) could be expressed to stimulate new blood vessel growth, and various new drug candidate molecules could be administered to the mammal, with the goal of testing whether such new drug candidate molecules inhibit new blood vessel growth stimulated by the rAAV-delivered angiogenic factor(s).

Expression of the heterologous nucleic acid sequence is under the control of a promoter/regulatory sequence. By "promoter/regulatory sequence" is meant a DNA sequence that is required for expression. In some instances, the promoter/regulatory sequence may be a core promoter sequence and in other instances, the promoter/regulatory sequence may also include an enhancer sequence and/or other regulatory sequences that enhance expression of the heterologous nucleic acid sequence. The promoter may be one that is constitutive or it may be inducible. If constant expression of the heterologous nucleic acid sequence is desired, then a constitutive promoter is used. Examples of well known constitutive promoters include the immediate-early cytomegalovirus (CMV) promoter, the Rous sarcoma virus promoter, and the like. Numerous other examples of constitutive promoters are well known in the art and can be employed in the practice of the invention.

If controlled expression of the heterologous nucleic acid sequence is desired, then an inducible promoter may be used. In an uninduced state, the inducible promoter is "silent." By "silent" is meant that little or no heterologous nucleic acid expression is detected in the absence of an inducer; in the presence of an inducer, however, heterologous nucleic acid expression occurs. Often, one can control the level of expression by varying the concentration of inducer. By controlling expression, for example by varying the concentration of an inducer so that an inducible promoter is stimulated more strongly or more wealdy, one can affect the concentration of the transcribed product of the heterologous nucleic acid sequence. In the case where the heterologous nucleic acid sequence codes for a gene, one can control the amount of protein that is synthesized. In this manner, it is possible to vary the concentration of therapeutic product. Examples of well known inducible promoters are: an estrogen or androgen promoter, a metallothionein promoter, or an ecdysone-responsive promoter. Numerous other examples are well known in the art and can be employed in the practice of the invention.

In addition to constitutive and inducible promoters (which tend to work in a wide variety of cell or tissue types), tissue-specific promoters can be used to achieve tissue- or cell-specific expression of the heterologous nucleic acid sequence. Well-known examples of tissue-specific promoters include several muscle-specific promoters including: skeletal α-actin promoter, cardiac actin promoter, skeletal troponin C promoter, the slow-twitch cardiac troponin C promoter, and the creatine kinase promoter/enhancer. There are numerous muscle-specific promoters that are well known in the art and can be employed in the practice of the invention (for a review on muscle-specific promoters see Miller et al. (1993) Bioessays 15:191-196).

Once delivered, the heterologous nucleic acid sequence, contained within the rAAV virion, is expressed to elicit a therapeutic effect By "therapeutic effect" is meant a level of expression of one or more heterologous nucleic acid sequences sufficient to alter a component of a disease (or disorder) toward a desired outcome or endpoint, such that a patient's disease or disorder shows improvement, often reflected by the amelioration of a sign or symptom relating to the disease or disorder.

The inventive use is also for treating ischemia in humans. The methods include the delivery of rAAV virions containing a heterologous nucleic acid sequence (i.e., a heterologous gene) encoding an angiogenic factor, the expression of which results in a therapeutic effect. The rAAV virions are introduced into a mammal by direct intramuscular injection into a muscle. In one preferred embodiment, the ischemic patient is injected at least once into muscle tissue with rAAV virions containing a heterologous nucleic acid sequence coding for one of the angiogenic factors. In one aspect, the therapeutic effect obtained is an increase in blood vessel formation. In another aspect, an increase in blood flow to the ischemic tissue is achieved. One skilled in the art will appreciate that other clinical parameters may be measured to determine whether a therapeutic effect was achieved.

The dose of rAAV virion required to achieve a particular therapeutic effect, e.g., the total number of rAAV virions introduced into the mammalian subject, will vary based on several factors including, but not limited to: the mammalian species, the route of rAAV virion administration, the level of heterologous nucleic acid sequence expression required to achieve a therapeutic effect, the specific disease or disorder being treated (e.g., peripheral or myocardial ischemia), a host immune response to the rAAV virion, a host immune response to the heterologous nucleic acid sequence expression product, and the stability of the expression product. One skilled in the art can readily determine a rAAV virion dose range to treat a patient having a particular disease or disorder based on the aforementioned factors, as well as other factors. With respect to treating an ischemic patient, a dose is provided that is at least 10¹⁰ rAAV virions, preferably between about 10¹⁰- 10¹⁵, more preferably between about 10¹¹ -10¹⁴, and most preferably between about 10¹²-10¹³ rAAV virions to achieve a desired therapeutic effect.

The invention provides methods for successful rAAV virion transduction leading to therapeutic expression of heterologous nucleic acid sequences. For the purposes of clarity and for exemplifying the best mode of the present invention, the discussion that follows exemplifies rAAV virion delivery of VEGF to a mammal.

Below are examples of specific embodiments for carrying out the present invention. The examples are offered for illustrative purposes only, and are not intended to limit the scope of the present invention in any way.

### EXAMPLE 1

### Recombinant AAV-hVEGF₁₆₅ Vector Construction, Production, and Purification

The recombinant adeno-associated virus human vascular endothelial growth factor₁₆₅ vector (rAA V-h VEGF₁₆₅) was constructed using standard molecular biology techniques that are well known in the art. Briefly, the beta-galactosidase gene (LacZ) was excised from the rAAV-LacZ vector (rAAV-LacZ is described in U. S. Patent No. 5,858,351) and replaced with full-length human VEGF₁₆₅ cDNA driven by the CMV promoter and flanked, on its 3'end, by the SV40 polyadenylation sequence. Recombinant AAV-h VEGF₁₆₅ virions were then produced using the triple transfection method described in U. S. Patent Nos. 6,001,650 and 6,004,797, supra, and purified using techniques also described in U. S. Patent Nos. 6,001,650 and 6,004,797, supra.

### EXAMPLE 2

### In Vitro AAV-hVEGF₁₆₅ Rat Cardiac Myocyte Assays

Cardiac myocytes were prepared from ventricles of 1-day-old Sprague-Dawley rats. After dissociation in 0.25% trypsin, cell suspensions were washed with DMEM (GIBCO BRL, Grand Island, NY) supplemented with 10% FCS (Cell Culture Laboratories, Cleveland, OH), and centrifuged at 500 g for 10 min. The cell pellets were then resuspended in DMEM containing 10% FCS. For selective enrichment of cardiac myocytes, the dissociated cells were pre-plated for 1 h, during which the non-myoctyes readily attached to the bottom of the culture dishes. The resulting suspensions of myocytes were plated onto 24-well dishes at a density of 4 x 10⁵ cells/well. Seventy-two hours after plating, the cultured rat cardiac myocytes were transduced with rAAV-hVEGF₁₆₅ virions (5 x 10³ virions/cell) for 24 h. To detect VEGF in the cytosol of transduced rat cardiac myocytes, immunoblotting was performed. Transduced rat cardiac myocytes were rinsed with ice-cold PBS and resuspended in lysis buffer (1% Nonidit P-40, 50 mM Tris-HCl, pH 7.4, 150 mM NaCl, 200 U/mL aprotinine, 1 mM PMSF). After incubation on ice for 30 min, cell extracts were centrifuged to remove cell debris. The cell lysates (15 µg of protein) were then separated by 7.5% polyacrylamide gel electrophoresis and blotted onto polyvinylidene difluoride membranes. The membranes were incubated for 1 h at room temperature in TBS with Tween 20 (TBST: 20 mM Tris-HCl, pH 7.4, 150 mM NaCl, 0.05% Tween 20) and 4% nonfat milk. The membranes were then incubated with anti-human VEGF₁₆₅ antibody (Sigma, St. Louis, MO) at 1:1000 dilution overnight at 4 °C in TBST. Specific binding of the antibody was visualized by the ECL detection system (Amersham, Buckinghamshire, UK) according to the manufacturer's instructions. In rAAV-hVEGF₁₆₅-transduced rat cardiac myocytes, VEGF was detected by immunoblot. Recombinant AAV-LacZ was used as a negative control; in rAAV-LacZ-transduced rat cardiac myocytes, VEGF was not detected (construction of the rAAV-LacZ vector is fully described in U. S. Patent No. 5,858,351, supra). FIG. 1 depicts the immunoblot results.

To measure the presence of VEGF in rat cardiac myocyte tissue, immunohistochemical staining was performed. Transduced rat cardiac myocytes were rinsed twice with TBS and blocked with normal rabbit serum, diluted 1:5 in TBS, for 45 min. The cells were incubated overnight with anti-human VEGF₁₆₅ antibody (2 µg/mL) followed by incubation for 1 h with rabbit anti-mouse IgG peroxidase conjugate (dilution, 1:50; preabsorbed overnight at 4 °C with 10% preimmune rat serum and 3 % bovine serum albumin). Antibody binding was visualized with 3,3-diaminobenzidine tetrahydrochloride (DAB, Sigma) in 0.1 M Tris buffer, pH 7.2, containing 0.01% H₂O₂. Negative control experiments consisted of omission of the vector from the incubation medium. FIG. 2 depicts the results. Approximately 60% of rAAV-hVEGF₁₆₅-transduced rat cardiac myocytes stained positive for VEGF (FIG. **2A**), whereas rAAV-LacZ-transduced rat cardiac myocytes did not show staining for VEGF (FIG. **2B**).

To measure VEGF secretion in the culture medium of transduced rat cardiac myocytes, enzyme-linked immunosorbent assay (ELISA) was performed in accordance with the manufacturer's instructions included in the ELISA kit (Amersham). After the myocytes had been incubated with rAAV-hVEGF₁₆₅ for 24 h, the myocytes were rinsed twice with PBS and incubated in FCS-free medium. Twenty-four hours after replacement of the culture medium, the concentration of VEGF in the medium was measured. FIG. 3 depicts the results. The VEGF concentration in the culture medium increased in a vector dose-dependent manner. Maximal concentration of secreted VEGF occurred at a vector dose of 5 x 10³ and was measured to be 6 ng/mL. The culture medium from non-transduced rat cardiac myocytes and rAAV-LacZ-transduced rat cardiac myocytes contained no detectable VEGF.

### EXAMPLE 3

### In Vivo Mouse rAAV-hVEGF₁₆₅ Ischemic Heart Assay

To produce ischemic myocardium, the trachea of an adult mouse is exposed through a midline incision of the neck, a tube is then placed in the trachea by using an appropriately-sized angiocatheter (such as one available from Becton Dickson), and the tube is then connected to a ventilator (such as the Small Animal Volume Controlled Ventilator available from Harvard Rodent Ventilator, model 683, Harvard Apparatus, South Natick, MA). After ventilator control of respiration is established, a thoracotomy incision is made in the second intercostals space, and a small retractor is placed in the incision to expose the heart. The anterior descending coronary artery is then ligated with surgical suture. Approximately 10¹⁰ to 10¹⁵ rAAV-hVEGF₁₆₅ virions contained in 50 µL of PBS are injected directly into several sites of the myocardium on the left ventricular wall around the ischemic region. After rAAV virions are inoculated, a small tube connected to a syringe is placed in the incision to evacuate air in the thoracic cavity, restoring negative pressure prior to the closing of the incision. The tube in the trachea is gently retracted after voluntary respiration is restored and the incision on the neck is then closed. Recombinant AAV-hVEGF₁₆₅ virions are injected into the myocardium at several sites around an ischemic region. Hearts are then collected 2 months after inoculation with rAAV-hVEGF₁₆₅, and compared to a control group of mice (i.e., mice not made surgically ischemic) that also receive the same dose of rAAV-hVEGF₁₆₅ virions delivered in the same manner. At least two hearts are collected per group of experimental animals. The numbers of small blood vessels are counted with a microscope using a x 20 objective.

### EXAMPLE 4

### Administration of AAV-LacZ Vectors to Rat Skeletal Muscle

Male Sprague-Dawley rats (200-250 g) were anesthetized with diethyl ether. A skin incision approximately 5 mm in length was made over the tibialis anterior muscle and the fascia identified. Recombinant AAV vectors were diluted in 50 mM Hepes- buffered saline and carbon black was added (Pelikan Ink, Gunther Wagner) to allow for tracing of the injection site. 200 µL of rAAV vector suspension containing AAV-LacZ (10¹³ virions/200 µL) were injected with a 29-gauge needle directly into the tibialis anterior muscle. X-Gal histochemical staining revealed β-galactosidase expression in the majority of the muscle fibers in the area of injection and this pattern remained consistent for the duration of the study period, which was 12 weeks.

### EXAMPLE 5

### Administration of rAAV-hVEGF₁₆₅ to Rat Ischemic and Contralateral Hindlimbs

Male Sprague-Dawley rats were anesthetized with an intraperitoneal injection of sodium pentobarbital (50 mg/kg). A longitudinal incision was made in the right thigh, after which the right femoral artery was surgically excised to induce limb ischemia. Rats were then transduced with rAAV-hVEGF₁₆₅ (2 x 10¹³ virions; n=8) via intramuscular injection at sites within the ischemic hindlimb and also at sites within the contralateral limb. The vector suspension (100 µL/site) was injected into 4 different sites in the major thigh muscles (quadriceps and adductor). Three rats received sham operations for preliminary assessment of hemodynamic examination. To confirm VEGF expression and to assess the possibility that VEGF was expressed in remote tissues, reverse transcription-polymerase chain reaction (RT-PCR) was performed. Gene expression at the mRNA level was evaluated by RT-PCR. Total cellular RNA of muscle tissues and remote tissues (e.g., brain, heart, liver, spleen, kidney, testes) was isolated using RNA STAT-60 (TET-TEST, Inc., Friendswood, Tex.). Extracted RNA was treated with DNase I (Takara Shuzo Co., Tokyo, Japan) to eliminate DNA contamination. The synthesis of first-strand cDNA was performed under conditions recommended in the ProSTAR First Strand RT-PCR kit (Stratagene, La Jolla, Calif.). The PCR amplifications were performed using human VEGF-specific primers (sense: 5'-GAGGGCAGAATCATCACGAAGT-3'; antisense: 5'-CCACCTTCTTGATGTCATCA-3'). GAPDH mRNA served as an internal standard. The PCR products were electrophoresed on ethidium bromide-stained 2.0% agarose gels. VEGF gene expression was observed 4 and 10 weeks after injection (FIG. 4 shows the results). On the other hand, no VEGF gene expression was detected in AAV-LacZ-transduced muscle. VEGF gene expression was not detected in the brain, heart, liver, spleen, kidney, and testes in rAAV-hVEGF₁₆₅-treated rats 4 weeks after injection (FIG. 4).

### EXAMPLE 6

### VEGF Secretion from Transduced Rat Skeletal Muscle

VEGF secretion from transduced ischemic hindlimb and contralateral hindlimb muscle was examined. Recombinant AAV-hVEGF₁₆₅-injected tibialis anterior muscle tissues were excised and cultured in serum free DMEM/F-12 medium. The ELISA kit was used to determine muscle culture supernatant VEGF concentrations. Ten weeks after rAAV-hVEGF165 injection, VEGF, up to 5.3 ± 1.5 ng/g tissue/24 h, was detected in the culture supernatant of rAAv-hVEGF₁₆₅ (1.8 x 10¹³ virions/site)-injected muscle tissue (FIG. 5). ELISA measurements did not reveal any VEGF in blood taken from the peripheral veins of the rats at 1, 2, 4, and 8 weeks after rAAV-hVEGF₁₆₅ injection.

### EXAMPLE 7

### Blood Flow Measurement and Thermography

Six weeks after rAAV-V-EGF₁₆₅ gene transfer, each rat was re-anesthetized with an intraperitoneal injection and the lower body coats were shaved. The skin temperature of the rat hindlimb was measured with infrared thermography (TH3106ME, NEC San-ei Instruments, Ltd., Tokyo, Japan). Blood flow at the tibialis posterior artery was measured by a transit-time ultrasound flowmeter (T206, Transonic Systems, Inc., Ithaca, New York) using a perivascular flowprobe (a representative graph is depicted in FIG. **6A**). The tibialis posterior artery was dissected free and perivascular flowprobes placed according to the manufacturer's instructions. Blood flow of both the ischemic and contralateral limbs was recorded simultaneously, and was expressed as a % of the contralateral limbs. Six weeks after AAV-LacZ and rAAV-hVEGF₁₆₅ injection, blood flow was measured. As shown in FIG. **6B**, the mean blood flow in rAAV-hVEGF₁₆₅-transduced ischemic hindlimbs (78.2 ± 11.3%) was significantly higher than that in AA.V-LacZ-transduced ischemic limbs (41.5 ± 5.4%). Measurements using infrared thermography showed comparable functional increases (representative thermographic images are depicted in FIG. **7A**). The skin temperature of the AAV-LacZ-transduced ischemic limb was approximately 2 °C lower than that of the contralateral limb. The skin temperature of the rAAV-hVEGF₁₆₅-transduced ischemic limb, however, was restored to a normal temperature (FIG. **7B**).

### EXAMPLE 8

### Histological Assessment

Six weeks after AAV-LacZ and rAAV-hVEGF₁₆₅ injection, ischemic muscle tissues were obtained as transverse sections from the quadriceps and adductor muscles of the rat ischemic hindlimb after hemodynamic examination. Frozen sections were stained with alkaline phosphatase using an indoxyl-tetrazolium method to detect capillary endothelial cells (FIG. **8A** is a representative image of rat muscle tissue using this method). Capillary density was evaluated by histological examination of 5 randomly selected fields of one muscle section, and the number of capillaries was counted (mean number of capillary per mm²). As shown in FIG. **8B**, capillary density was significantly higher in rAAV-hVEGF165-injected muscle (1062 ± 75/mm²) than that in AAV-LacZ-injected muscle tissues (532 + 24/mm²). There was no angiomalike structures or inflammatory-cell infiltration observed in either ischemic or contralateral limbs.

## Claims

1. Use of recombinant adeno-associated virus (rAAV) virions free of wild-type AAV virions and helper-virus, comprising a gene encoding an angiogenic factor in the manufacture of a medicament for the treatment of ischemic conditions, wherein the rAAV virions are introduced via direct injection into a muscle.

2. The use of claim 1, wherein the angiogenic factor is selected from the group consisting of fibroblast growth factor (FGF), angiopoietin-1, and vascular endothelial growth factor (VEGF).

3. The use of claim 2, wherein the angiogenic factor is a vascular endothelial growth factor (VEGF).

4. The use of claim 3, wherein said VEGF is VEGF165.

5. The use of claims 1 to 4, wherein about 10¹⁰ to about 10¹⁵ rAAV virions are delivered.

6. The use of claims 1 to 5, wherein at least two angiogenic factor genes are delivered.

7. The use of claim 6, wherein a gene coding for VEGF and a gene coding for angiopoietin-1 are delivered by said rAAV virions.

8. The use of claim 6, wherein a gene coding for FGF and a gene coding for VEGF are delivered by said rAAV virions.

9. The use of any of claims 1 to 8, wherein said muscle is a cardiac muscle.

10. The use of any of claims 1 to 8, wherein said muscle is a skeletal muscle.

11. The use of any of claims 1 to 8, wherein said muscle is a smooth muscle.

12. The use of any of claims 1 to 11, wherein said treatment involves the formation of new blood vessels.

13. The use of any of claims 1 to 11, wherein said treatment involves an increase in blood flow.

## Patentansprüche

1. Verwendung rekombinanter Adeno-assoziierter Virus (rAAV) Virions, die von Wildtyp AAV-Virions und Helfervirus frei sind, umfassend ein Gen, codierend einen angiogenen Faktor bei der Herstellung eines Medikaments für die Behandlung ischämischer Zustände, wobei die rAAV-Virions über direkte Injektion in einen Muskel eingeführt werden.

2. Verwendung gemäß Anspruch 1, wobei der angiogene Faktor ausgewählt ist aus der Gruppe bestehend aus Fibroblasten-Wachstumsfaktor (FGF), Angiopoietin-1 und vaskulärem endothelialem Wachstumsfaktor (VEGF).

3. Verwendung gemäß Anspruch 2, wobei der angiogene Faktor ein vaskulärer endothelialer Wachstumsfaktor (VEGF) ist.

4. Verwendung gemäß Anspruch 3, wobei das VEGF VEGF165 ist.

5. Verwendung gemäß den Ansprüchen 1 bis 4, wobei ungefähr 10¹⁰ bis ungefähr 10¹⁵ rAAV-Virions zugeführt werden.

6. Verwendung gemäß den Ansprüchen 1 bis 5, wobei mindestens zwei angiogene Faktorgene zugeführt werden.

7. Verwendung gemäß Anspruch 6, wobei ein für VEGF codierendes Gen und ein für Angiopoietin-1 codierendes Gen durch die rAAV-Virions zugeführt werden.

8. Verwendung gemäß Anspruch 6, wobei ein für FGF codierendes Gen und ein für VEGF codierendes Gen durch die rAAV-Virions zugeführt werden.

9. Verwendung gemäß einem der Ansprüche 1 bis 8, wobei der Muskel ein Herzmuskel ist.

10. Verwendung gemäß einem der Ansprüche 1 bis 8, wobei der Muskel ein Skelettmuskel ist.

11. Verwendung gemäß einem der Ansprüche 1 bis 8, wobei der Muskel ein glatter Muskel ist.

12. Verwendung gemäß einem der Ansprüche 1 bis 11, wobei die Behandlung die Bildung neuer Blutgefäße involviert.

13. Verwendung gemäß einem der Ansprüche 1 bis 11, wobei die Behandlung einen Anstieg im Blutfluß involviert.

## Revendications

1. Utilisation de virions d'un virus associé aux adénovirus recombinant (rAAV) exempts de virions AAV de type sauvage et d'un virus auxiliaire, comprenant un gène codant un facteur angiogénique dans la fabrication d'un médicament destiné au traitement des états ischémiques, dans laquelle les virions rAAV sont introduits via une injection directe dans un muscle.

2. Utilisation selon la revendication 1, dans laquelle le facteur angiogénique est choisi dans le groupe constitué du facteur de croissance des fibroblastes (FGF), de l'angiopoiétine-1, et du facteur de croissance endothélial vasculaire (VEGF).

3. Utilisation selon la revendication 2, dans laquelle le facteur angiogénique est un facteur de croissance endothélial vasculaire (VEGF).

4. Utilisation selon la revendication 3, dans laquelle ledit VEGF est le VEGF165.

5. Utilisation selon les revendications 1 à 4, dans laquelle environ 10¹⁰ à environ 10¹⁵ virions rAAV sont délivrés.

6. Utilisation selon les revendications 1 à 5,`dans laquelle au moins deux gènes de facteur angiogénique sont délivrés.

7. Utilisation selon la revendication 6, dans laquelle un gène codant pour le VEGF et un gène codant pour l'angiopoiétine-1 sont délivrés par lesdits virions rAAV.

8. Utilisation selon la revendication 6, dans laquelle un gène codant pour le FGF et un gène codant pour le VEGF sont délivrés par lesdits virions rAAV.

9. Utilisation selon l'une quelconque des revendications 1 à 8, dans laquelle ledit muscle est un muscle cardiaque.

10. Utilisation selon l'une quelconque des revendications 1 à 8," dans laquelle ledit muscle est un muscle squelettique.

11. Utilisation selon l'une quelconque des revendications 1 à 8, dans laquelle ledit muscle est un muscle lisse:

12. Utilisation selon l'une quelconque des revendications 1 à 11, dans laquelle ledit traitement comprend la formation de nouveaux vaisseaux sanguins.

13. Utilisation selon l'une quelconque des revendications 1 à 11, dans laquelle ledit traitement comprend une augmentation du flux sanguin.
